# EUROPEAN PATENT APPLICATION

(11) **EP 1 580 554 A1**
(43) Date of publication of application: **28.09.2005**
(21) Application number: 03812332.9
(22) Date of filing: 28.11.2003
(51) Int. Cl.: G01N 33/53, C12N 15/09, C12M 1/00, C12Q 1/68

(54) **METHOD OF IMMOBILIZING POLYNUCLEOTIDE ON SOLID SUPPORT, SOLID IMMOBILIZATION SUPPORT THUS OBTAINED, METHOD OF DETECTING TARGET POLYNUCLEOTIDE FROM THE SOLID SUPPORT AND SOLUTION FOR IMMOBILIZING POLYNUCLEOTIDE**

(30) Priority: 02.12.2002 JP 2002350225
(71) Applicant: Toyo Kohan Co., Ltd., Tokyo 102-8447 (JP)
(72) Inventor: TANGA, M. TOYO KOHAN CO., LTD. Technical Res. Lab., Yamaguchi 744-8611 (JP); OKAMURA, H. TOYO KOHAN CO., LTD. Techn. Res. Lab., Yamaguchi 744-8611 (JP); YAMANO, H. TOYO KOHAN CO., LTD. Techn. Res. Lab., Yamaguchi 744-8611 (JP); OHBA, M. TOYO KOHAN CO., LTD. Technical Res. Lab., Yamaguchi 744-8611 (JP); KAMEI, S. TOYO KOHAN CO., LTD. Technical Res. Lab., Yamaguchi 744-8611 (JP); ICHIHARA, T. TOYO KOHAN CO., LTD. Tech. Res. Lab., Yamaguchi 744-8611 (JP)
(74) Representative: Vossius & Partner
(86) International application number: PCT/JP2003/015220
(87) International publication number: WO 2004/051272

(57) **Abstract**

It is intended to provide a method of immobilizing a polynucleotide on a solid support by spotting the polynucleotide on the solid support in a uniform spot shape without any irregularity. Namely, a method of immobilizing a polynucleoiide on a solid support by spotting a solution containing the polynucleotide together with polyether or glycol on the solid support.

## Description

### Technical Field

The present invention concerns a method of immobilizing a polynucleotide on a solid support which is useful for the preparation of DNA chips, etc. Further, the present invention concerns a polynucleotide immobilized solid support prepared by the method, a method of detecting a polynucleotide by using the same, and a solution for immobilizing the polynucleotide.

### Background Art

Attempts have been made not only for making the gene structures of versatile living matters but also for analyzing the gene functions thereof at the genome scale, and development for the technique of efficiently analyzing the gene functions has also been proceeded rapidly. A DNA micro-array (also referred to as DNA chip) is a high density array in which plural DNA molecules are arranged and immobilized on every predetermined regions to a substrate such as of slide glass, which is extremely useful for the determination of base sequences of nucleic acids, as well as simultaneous analysis, for example, of gene expression, variant and polymorphism. Since the analysis for the gene information using the DNA micro-array is extremely useful, for example, in the drug creation and development for diagnostic and preventive methods of diseases, a further development has been desired for the technique of manufacturing the DNAmicro-array and the analysis system for the obtained data.

For detecting a nucleic acid such as DNA by using a DNA micro-array, a specimen of nucleic acid fragments (target nucleic acid sample) labelled with radio isotope (RI) or fluorescence is at first hybridized to a probe, for example, of DNA fragments arranged at high density on the surface of a solid support. In this case, molecules in the target nucleic acid having a base sequence complementary with the probe are hybridized in complementary to the probe. Then, signals of a hybridized labelled target molecule are measured to identify the hybridized probe polynucleotide. Specifically, the radiation intensity or florescence intensity on the micro-array is measured by an RI or florescence image scanner and the obtained image data are analyzed. Therefore, according to the DNA micro-array, from several thousands to several tens of thousands of molecules can be hybridized and detected simultaneously and a great amour of gene information can be obtained by using a slight amount of specimens in a short period of time.

Preparation of the DNA micro-array includes a method of synthesizing an oligonucleotide directly on the surface of a solid support (referred to as "on-chip method") and a method of immobilizing a previously prepared DNA fragment to the surface of the solid support. The former, on-chip method is a method of using a protective group to be removed selectively by photo-irradiation and conducting combinatorial synthesis with a number of fine matrixes on the solid support by utilizing the photolithography used in the manufacture of semiconductors and a solid phase synthesis technique, thereby synthesizing plural kinds of oligonucleotides simultaneously (Fodor, S.P.A. et al, Science, 251, 767-773 (1991)).

On the other hand, the latter method is a method of spotting previously prepared probe nucleotides such as a specimen of DNA fragments to the surface of a solid support thereby bonding and immobilizing the same by utilizing covalent bonding or ionic bonding. Such a method includes, for example, a method of spotting a probe polynucleotide to the surface of a solid support surface-treated with polycation (polylycine, polyethyleneimine, etc.) by using a spotting device provided to a DNA micro-array manufacturing apparatus and electrostatically coupling and immobilizing the same to the solid support by utilizing charges of the polynucleotide specimen (Schene, M. et al., Science, 270, 467-470 (1995)), a method of previously synthesizing an oligonucleotide introduced with a reactive group, spotting the same to the surface of a surface-treated solid support by using a spotting device and immobilizing the same by way of a covalent bond ("Protein - Nucleic Acid-Enzyme", 43, 2004-2011 (1998); Lamture, J.B. et al., Nucl. Acids Res., 22, 2121-2125 (1994); Guo, Z. et al., Nucl. Acids Res., 22, 5456-5465 (1994)), as well as a method of introducing a reactive group such as an active ester group that forms a covalent bond with a polynucleotide, and immobilizing a polynucleotide by way of a covalent bond (pamphlet of International Publication No. WO00/22108, pamphlet of International Publication No. WO01/75447 and pamphlet of International Publication No. WO02/12891), etc.

For forming plural spots on the surface of the solid support by immobilizing the probe polynucleotides, various types of spotting devices of a pin system of mechanically contacting a pin tip to the surface of a solid support, an ink jet system utilizing the principle of an ink jet printer, etc. have been used. The thus immobilized spots frequently show scattering in view of the shape. For example, a spot is in a crescent shape or doughnut shape, or spot is bleeded. In a case where the shape of the spot is not determined, the signal intensity also varies to lower the reliability of the data. Accordingly, for enhancing the detection accuracy as much as possible for the specimen of the nucleic acid fragment as an object of detection thereby improving the accuracy upon analysis by the micro array, it is desirable for the spot formed on the solid support that the probe molecules are firmly immobilized to the surface of the solid support and the shape and the size thereof are made uniform as much as possible and the reproducibility is favorable.

The subject of the present invention is to provide a method of immobilizing a polynucleotide on a solid support with no unevenness and in a uniform spot shape upon spotting of a polynucleotide to the solid surface.

### Disclosure of the Invention

The present inventors have made an honest study for solving the subject described above and, as a result, have found that a uniform spot can be obtained with no unevenness by dissolving a polynucleotide in a solution containing a polyether or glycol and spotting the obtained solution on a solid support, to accomplish the present invention.

That is, the present invention includes the following inventions.
(1) A method of spotting a solution containing a polyether or glycol and a polynucleotide to a solid support thereby immobilizing the polynucleotide on the solid support.
(2) A method as described in (1) in which the solid support has a functional group capable of covalent-bonding with a polynucleotide.
(3) A method as described in (2) in which the solid support has an electrostatic layer for electrostatically attracting the polynucleotide on a substrate.
(4) A method as described in any one of (1) to (3) in which the polynucleotide is DNA.
(5) A method as described in any one of (1) to (4) in which the polyether or glycol is diethylene glycol or polyethylene glycol.
(6) A solid support in which a polynucleotide is immobilized by the method as described in any one of (1) to (5).
(7) A method of detecting a target polynucleotide, which includes hybridizing a labelled polynucleotide to a polynucleotide immobilized on a solid support by the method as described in any one of (1) to (5) and reading a signal derived from the labelled polynucleotide.
(8) A solution for immobilizing a polynucleotide characterized in that it contains a polyether or glycol.

### Brief Description of the Drawings

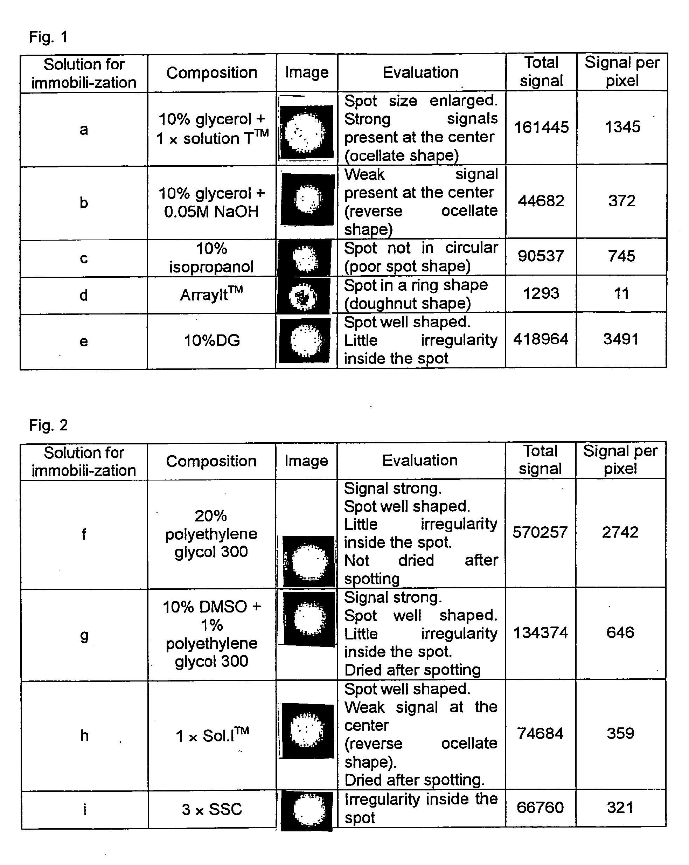

Fig. 1 shows fluorescence images obtained by spotting and incubating fluorescence labelled DNA solutions of various compositions on a solid support and then photographing them by using FLA8000. Fig. 2 shows fluorescence images obtained by spotting and incubating fluorescence labelled DNA solutions of various compositions on a solid support and then photographing them by using FLA8000.

### Best Mode for Practicing the Invention

Description is to be made to a method of immobilizing a probe polynucleotide such as a DNA fragment on a solid support in the present invention.

In the present invention, the solid support to immobilize the polynucleotide is not particularly limited and those having an electrostatic layer for electrostatically attracting the polynucleotide on a substrate, and a functional group capable of covalent-bonding with a polynucleotide are preferred.

The material for the substrate used in the present invention includes, for example, silicon, glass, fiber, wood material, paper, ceramics, plastics (for example, polyester resin, polyethylene resin, polypropylene resin, ABS resin(acrylonitrile butadiene styrene resin), nylon, acryl resin, fluoro resin, polycarbonate resin, polyurethane resin, methylpentene resin, phenol resin, melamine resin, epoxy resin, and vinylchloride resin).

In a case of using those described above as the material for the substrate, while a surface treatment layer may not be applied but application of the surface treatment is more preferred for strongly immobilizing, on the substrate, a compound for introducing a functional group capable of covalent-bonding with a polynucleotide.

It is preferred for the surface treatment to use any one of synthetic diamond, high pressure synthetic diamond, natural diamond, soft diamond (for example, diamond-like carbon), amorphous carbon, carbonaceous material (for example, graphite, fullerene, or carbon nano-tube), a mixture thereof or a laminate thereof. Further, carbides such as hafnium carbide, niobium carbide, silicon carbide, tantalum carbide, thorium carbide, titanium carbide, uranium carbide, tungsten carbide, zirconium carbide, molybdenum carbide, chromium carbide and vanadium carbide may also be used. The soft diamond collectively means incomplete diamond structures which are mixtures of diamond and carbon such as so-called diamond-like carbon (DLC), in which the mixing ratio thereof is not particularly limited.

Examples of the surface treated substrate includes a substrate in which a film of soft diamond is formed to slide glass. such a substrate is preferably those in which diamond-like carbon is prepared in a gas mixture containing 0 to 99 % by volume of a hydrogen gas and remaining 100 to 1 % by volume of a methane gas by an ionizing vapor deposition method.

The thickness of the surface treatment layer is preferably form 1 nm to 100 µm.

The surface treatment layer for the substrate can be formed by a known method, for example, a microwave plasma CVD (Chemical Vapor Deposit) method, an ECRCVD (Electric Cyclotron Resonance Chemical Vapor Deposit) method, an ICP (Inductive Coupled Plasma) method, a direct current sputtering method, an ECR (Electric Cyclotron Resonance) sputtering method, an ion plating method, an arc ion plating method, an EB (Electron Beam) vapor deposition method, an ohmic heating vapor deposition method, an ionizing vapor deposition method, an arc vapor deposition method, a laser vapor deposition method, etc.

The substrate used in the present invention includes not only those structures formed with the surface treatment layer as described above, but also those obtained from synthetic diamond, high pressure synthetic diamond, natural diamond, soft diamond (for example, diamond-like carbon), amorphous carbon; metals such as gold, silver, copper, aluminum, tungsten and molybdenum; plastics (for example, polyester resin, polyethylene resin, polypropylene resin, ABS resin, nylon, acryl resin, fluoro resin, polycarbonate resin, polyurethane resin, methylpentene resin, phenol resin, melamine resin, epoxy resin and vinylchloride resin); those formed by mixing and bonding metal powder and ceramic powder described above with resin described above as a binder; or materials such as the metal powder or the ceramics powder described above compacted by a press molding machine, which are sintered at high pressure. Further, they may be a laminate or a composite body of the materials (for example, composite material of diamond and other substance (for example, 2-phase body)).

The shape and the size of the substrate is not particularly limited and the shape can include, for example, plate shape, thread shape, spherical shape, polygonal shape and powdery shape. In a case of using a plate, it is usually about from 0.1 to 100 mm width, 0.1 to 100 mm of length and 0.01 to 10 mm thickness.

Further, a single layer made of Ti, Au, Pt, Nb, Cr, TiC, TiN, etc. or a composite film thereof may be formed as a reflection layer to the surface or the rear face of the substrate. Since the thickness of the reflection layer has to be uniform entirely, it is preferably 10 nm or more and more preferably, 100 nm or more.

The solid support in the invention is preferably provided with an electrostatic layer for electrostatically attracting the polynucleotide.

The electrostatic layer has no particular restriction so long as it electrostatically attracts the polynucleotide and improves the immobilized amount of the polynucleotide and, it can be formed, for example, by using a compound having a positive charges such as an amino group containing compound.

The amino group containing compound includes those compounds having a non-substituted amino group (-NH₂) or a mono-substituted amino group with an alkyl group of 1 to 6 carbon atoms (-NHR: R being substituent), for example, ethylene diamine, hexamethylene diamine, n-propyl amine, mono-methyl amine, dimethyl amine, monoethyl amine, diethyl amine, allyl amine, amino azobenzene, amino alcohol (for example, ethanol amine), acrinole, aminobenzoic acid, aminoanthraquinone, amino acid (glycin, alanine, valine, leucine, serine, threonine, cystine, methionine, phenylalanine tryptophan, tyrosine, prolyne, cistine, glutamic acid, aspartic acid, glutamine, asparagine, lysine, arginine, and histidine), aniline, or polymer thereof (for example, polyarylamine, polylysine) and copolymer thereof, and polyamine (polyvalent amines) such as 4,4',4"-triaminotriphenylmethane, triamterene, spermidine, spermine and putrescine.

The electrostatic layer may be formed not by covalent bonding with the substrate or the surface treatment layer, or it may be formed by covalent bonding with the substrate or the surface treatment layer.

In a case of forming the electrostatic layer not by covalent bonding with the substrate or the surface treatment layer, an amino group-containing carbonaceous film is formed, for example, by introducing the amino group containing compound into a film forming apparatus upon forming the film of the surface treatment layer. As the compound to be introduced into the film forming apparatus, an ammonia gas may also be used. Further, the surface treatment layer may also be a composite layer formed such as by forming a close adhesion layer and then forming a film containing an amino group, which may also be conducted in an atmosphere containing an ammonia gas. Further, in a case of forming the electrostatic layer not by covalent bonding with the substrate or the surface treatment layer, it is preferred to vapor deposit the compound having the non-substituted or mono-substituted amino group and a carbon compound on the substrate and then introduce a functional group capable of covalent bonding with the polynucleotide. The carbon compound used herein has no particular restriction so long as it can be supplied as a gas and, for example, methane, ethane or propane which is gaseous at a normal temperature is preferred. The vapor deposition method is, preferably, an ionizing vapor deposition method, and the conditions for the ionizing vapor deposition method are preferably within a range for the operation pressure of 0.1 to 50 Pa and at the acceleration voltage of from 200 to 1000V.

In a case of forming the electrostatic layer by covalent bonding with the substrate or the surface treatment layer, the electrostatic layer can be form, for example, by irradiating UV-rays in a chlorine gas to a substrate or a substrate applied with a surface treatment layer thereby chlorinating the surface thereof and then reacting the amino group containing compound described above, for example, a polyvalent amine such as polyallyl amine, polylysine, 4,4', 4''- triaminotriphenylmethane, triamterene, etc. thereby introducing the amino group to the terminal on the side not bonded with the substrate. The amino group may also be introduced by chlorinating the surface of the substrate and then radiating UV-rays in an ammonia gas.

Further, in a case of conducting the reaction of introducing the functional group capable of covalent bonding with the polynucleotide to the substrate applied with the electrostatic layer (for example, introduction of a carboxylic group using a dicarboxylic acid or polybasic carboxylic acid) in a solution, it is preferred to immerse the substrate in a solution containing the compound having the non-substituted or mono-substituted amino group and then introduce the functional group capable of covalent bonding with the polynucleotide. The solvent for the solution includes, for example, water, N-methylpyrrolidone and ethanol.

In a case of introducing the carboxylic group by using a dicarboxylic acid or a polybasic carboxylic acid to the substrate applied with the electrostatic layer, this is preferably conducted by previous activation with N-hydroxyl succinimide and/or carbodiimides, or by conducting the reaction under the presence of N-hydroxy succinimide and/or carbodiimides.

When a polyallyl amine is used as the amino group containing compound in a case of forming the electrostatic layer by immersing the substrate in a solution containing the compound having a non-substituted or mono-substituted amino group, close bondability with the substrate is excellent further improve the immobilized amount of the polynucleotide.

The thickness of the electrostatic layer is preferably from 1 nm to 500 µm.

As described above, chemical modification is applied to the surface of the substrate applied with the electrostatic layer in order to introduce the functional group capable of covalent bonding with the polynucleotide.

The functional group includes, for example, a carboxylic group, active ester group, haloformyl group, hydroxyl group, sulfate group, cyano group, nitro group, thiol group and amino group. The compound used for introducing the carboxyl group as the functional group includes, for example, a halocarboxylic acid represented by the formula: X-R¹ -COOH (in which X represents a halogen atom, and R¹ represents a bivalent hydrocarbon group of 1 to 12 carbon atoms), for example, chloroacetic acid, fluoroacetic acid, bromoacetic acid, iodoacetic acid, 2-chloropropionic acid, 3-chloropopionic acid, 3-choroacrylic acid, and 4-chlorobenzoic acid; a carboxylic acid represented by the formula: HOOC-R²-COOH (in which R² represents a single bond or a bivalent hydrocarbon group of 1 to 12 carbon atoms), for example, oxalic acid, malonic acid, succinic acid, maleic acid, fumaric acid, and phthalic acid; a polybasic carboxylic acid such as polyacrylic acid, polymethaclyric acid, trimellitic acid, and butane tetracarboxylic acid; a keto acid or aldehyde acid represented by the formula: R³-CO-R⁴-COOH (in which R³ represents a hydrogen atom or a bivalent hydrocarbon group of 1 to 12 carbon atoms, R⁴ represents a bivalent hydrocarbon group of 1 to 12 carbon atoms); a monohalide of dicarboxylic acid represented by the formula: X-OC-R⁵-COOH (in which X represents a halogen atom, and R⁵ represents a single bond or a bivalent hydrocarbon group of 1 to 12 carbon atoms), for example, succinic acid monochloride and malonic acid monochloride; and an acid anhydride such as phthalic acid anhydride, succinic acid anhydride, oxalic acid anhydride, maleic acid anhydride, and butane tetracarboxylic anhydride.

The carboxylic group introduced as described above can be formed into an active ester by a dehydrating agent such as cyanamide or carbodimide (for example, 1-[3-(demethylamino) propyl]-3-ethyl carbodimide) and a compound such as N-hydroxyl succinimde.

The compound used for introducing the haloformyl group as the functional group includes, for example, a dihalide of a dicarboxylic acid represented by the formula: X-OC-R⁶-CO-X (in which X represents a halogen atom and R⁶ represents a single bond or a bivalent hydrocarbon group of 1 to 12 carbon atoms), for example, succinic acid chloride and malonic acid chloride.

The compound used for introducing the hydroxy group as the functional group includes, for example, a hydroxy acid represented by the formula: HO-R⁷-COOH (in which R⁷ represents a bivalent hydrocarbon group of 1 to 12 carbon atoms) or phenolic acid.

The compound used for introducing the amino group as the functional group includes, for example, amino acid.

The carboxyl group of the compound described above is condensed with the amino group in the electrostatic layer to form an amide coupling.

Among the compounds described above, the polybasic carboxylic acid such as polyacrylic acid, polymethacrylic acid, trimellitic acid, and butane tetracarboxylic acid can be used for improving the hydrophilicity.

In the step described above, the treatment with the amino group-containing compound, the treatment of introducing the functional group or the treatment of forming the active ester is preferably conducted repeatedly for several times.

The polynucleotide immobilized to the solid support to form a probe in the invention also includes oligonucleotides which include DNA and RNA. The polynucleotide may have a single strand or a double strand. For gene examining the gene expression, DNA fragments (polynucleotide) such as of cDNA, a portion of cDNA or EST are generally used. The sequence and the function of the polynucleotide may be unknown but generally they are prepared by using cDNA or genome library as a template, based on the sequence registered in the data base, by amplification according to the PCR method (herein after referred to as "PCR product"). Those not amplified by the PCR method may also be used.

Meanwhile, for examining gene variants or polymorphs, various polynucleotides corresponding to variants and polymorphs may be synthesized and used based on the known sequences as the standard. Further, in the case of determining the base sequence, 4n (n: base length) kinds of polynucleotides are synthesized and used. It is preferred that the base sequences of the polynucleotides used are already known. Usually, the polynucleotide to be immobilized is, preferably, from 2 to 200 mer and, particularly preferably from 10 to 120 mer.

Further, the polynucleotides in the invention also include artificial nucleic acids formed by converting the phospho-diester bond of DNA into the peptide bond, that is, peptide nucleic acid (PNA).

The probe polynucleotide described above is dissolved or dispersed in a solvent containing a polyether or glycol to prepare a solution for immobilizing the polynucleotide. The solvent containing the polyether or glycol includes both the solvent containing either the polyether or glycol and a solvent containing both of the polyether and glycol.

The glycol in the invention means a dihydric alcohol containing two hydroxy groups. In the invention, the glycol is preferably a dihydric linear alcohol, a dihydric linear alcohol having from 1 to 3,000,000 oxygen atoms in the main chain is more preferred and a dihydric linear alcohol having 1 to 30,000 ether bonds in the main chain is particularly preferred. It is considered that the glycol is a favorable solvent for the aim of immobilization since it does not hinder the reaction between the functional group on the substrate and the substance to be immobilized (DNA, etc.).

Specific example of the glycol includes, an alkylene glycol, a dialkylene glycol and a trialkylene glycol with the dialkylene glycol being preferred. The alkylene group is an alkylene group of 2 to 6,000,000 carbon atoms, preferably, 2 to 60,000 carbon atoms, for example, ethylene, propylene and butylene. In the present invention, use of dietylene glycol as the glycol is particularly preferred. Glycols may be used each alone or plural species of them may be used in admixture.

The concentration of the glycol in the solution is usually from 5 to 30% by weight, preferably, from 5 to 20% by weight and, more preferably, from 5 to 15% by weight. The glycol solution is usually at pH 4 to 12 and, preferably, at pH 6 to 8.

The polyether in the present invention means a linear high molecular polyether having ether bonds in the main chain. The polyether includes, for example, polyethylene glycol, a polyalkylene oxide such as polytrimethylene oxide, polybutene oxide and polypentene oxide, as well as polyoxymethylene. The polyalkylene oxide is preferred and the polyethylene glycol is particularly preferred. Further, a polyether with an average molecular weight of 100 to 2, 000, 000 is preferred and a polyether with an average molecular of 200 to 20, 000 is more preferred. Particularly, a polyethylene glycol with an average molecular weight of 200 to 2000 is preferred and a polyethylene glycol with an average molecular weight of 200 to 400 is more preferred. In the present specification, the average molecular weight means the weight average molecular weight. It is considered that the polyether is a preferred solvent for the aim of immobilization, since it does not hinder the reaction between the functional group on the substance and the substance to be immobilized.

In the present invention, the polyethylene glycol also includes a monoalkyl ether of polyethylene glycol and an alkyl group of 1 to 50 carbon atoms, for example, polyethylene glycol monodecyl ether and polyethylene glycol monooctyl ether. Further, in the present invention, a polyethylene glycol not in the form of the monoalkyl ether in which hydroxyl groups are present on both terminals is included also in the glycol. The polyethers may be used each alone or plural kinds of them may be used in admixture.

Specifically, they include polyethylene glycol 200 (average molecular weight of 190 to 200), polyethylene glycol 300 (average molecular weight of 285 to 315), polyethylene glycol 400 (average molecular weight of 380 to 420), polyethylene glycol 600 (average molecular weight of 570 to 630), polyethylene glycol 1000 (average molecular weight of 950 to 1050), polyethylene glycol 1500 (equi-amount mixture of PEG 300 and PEG 1540, average molecular weight of from 500 to 600), polyethylene glycol 1540 (average molecular weight of 1300 to 1600), polyethylene glycol 2000 (average molecular weight of 1800 to 2200), polyethylene glycol 4000 (average molecular weight of 2700 to 3400), polyethylene glycol 6000 (average molecular weight of 7400 to 9000), polyethylene glycol 20000 (average molecular weight of 18000 to 25000), polyethylene glycol 300 monodecyl ether, polyethylene glycol 600 monooctyl ether, and a mixture thereof. The polyethylene glycol with an average molecular weight of 150 to 450, for example, polyethylene glycol 200, polyethylene glycol 300, polyethylene glycol 400 and a mixture thereof advantageous since they are liquid at a room temperature and detection by visual observation, CCD (Charge Coupled Device) and cooled CCD is possible after spotting, defects such as spot leakage, etc. can be found easily.

The concentration of the polyether in the solution is usually from 1 to 100% by weight, preferably, 1 to 80% by weight and, more preferably, 5 to 40% by weight. The polyether solution is usually at pH 2 to 13, preferably, at pH 5 to 9.

The solvent for dissolving the polyether or glycol has no particular restriction and one or more of solvents selected from aqueous solvents, for example, distilled water and SSC (saline-sodium citrate), etc. and polar organic solvents capable of dissolving DNA, etc., for example, dimethyl sulfoxide, diethyl formamide, tetrahydro furan, trifluoroacetic acid, triethylamine, 1-methyl-2-pyrrolidone, dioxane, and ethyl acetate can be used. Co-existence of dimetyl sulphoxide is advantageous since the viscosity of the solution can be controlled while keeping the spot shape and the immobilizing force of the biological substances such as DNA to the solid support.

Further, the solvent may also contain alcohol, for example, monohydric alcohol such as methanol, ethanol, propanol and butanol, as well as ethylene glycol monoethyl ether and tripropylene glycol monomethyl ether, etc.

The polynucleotide solution is distributed to a plastic plate such as of 96-hole or 384-hole type, and a distributed aqueous liquid is dripped onto the solid support by using a spotting device, etc. to conduct spotting. As the spotting device, a pin-system device in which a probe polynucleotide solution is held to the pin, the pin is brought into contact with the surface of the solid support, and the aqueous liquid is transferred to the surface of the solid support to form a spot is usually used. The shape at the top end of the pin includes various types such as a solid pin type (not grooved particularly) and quill pin type (grooved as in a fountain pen), both of which can be used. The quill pin type is preferred. Further, in addition to the pin system, an inkjet system utilizing the principle of an inkjet printer, or a spotting device utilizing a capillary system by means of a capillary tube may also be used.

The polynucleotide to be spotted is preferably within a range from 10² to 10⁵ types/cm² relative to the surface of the solid support. The amount of the probe polynucleotide is in a rage from 1 to 10⁻¹⁵ mol and the weight is preferably several ng or less. By the spotting, the probe polynucleotide is immobilized as a spot to the surface of the solid support. By the use of the method according to the present invention, the shape for each of the spots is substantially circular and of substantially an identical size, and uniform spots are formed with no blanking. It is important for the quantitative analysis of the gene expression or analysis for the single base variant that the shape or the size does not fluctuate.

The spotting amount of the solution per spot is usually within a range from 1 pL to 1 µL and, preferably, within a range from 100 pL to 100 nL. The size of the spot is usually within a range of the diameter from 50 to 300 µm. Then the distance between the spots is usually within a range of 0 to 1.5 mm and, preferably, within a range from 100 to 700 µm.

After the spotting of the polynucleotide solution, the solid support is preferably incubated usually at a room temperature to 120°C, preferably, at 60 to 100°C and, usually, for 72 hours or less and, preferably, for 1 to 3 hours. By applying the post treatment described above, stable covalent bond is formed between the polynucleotide and the solid support.

After immobilizing the polynucleotide to the solid support, the solid support is cleaned for removing not immobilized polynucleotide, etc. As the cleaning solution, those used customarily in the relevant technical field can be used and, for example, an aqueous solution containing 2 × SSC, 0.2% SDS (sodium dodecyl sulfate) can be used.

The solid support to which the polynucleotide is immobilized of the present invention prepared by the method of immobilizing the polynucleotide has a number of spots (usually from several hundreds to several tens of thousands) comprising regions in which a number of probe polynucleotides are immobilized by the covalent bond.

The working life of the micro-array prepared in accordance with the immobilizing method for the polynucleotide is usually from 2 to 6 months for the cDNA analyzing micro-array in which cDNA is immobilized and from 2 to 6 months for the oligo-DNA analyzing micro-array in which oligo-DNA is immobilized. The DNA analyzing micro-arrays are utilized for monitoring of gene expression, determination of base sequence, analysis for variant, analysis for polymorphism, etc.

Then, the detection method for the polynucleotide specimen using the solid support according to the present invention is to be described. At first, a specimen for labelled polynucleotide is provided. As the polynucleotide specimen (target polynucleotide), a DNA fragment or RNA fragment with unknown sequence and function is usually used.

Generally, for the purpose of examining the gene expression, generally, the labelled polynucleotide specimen is obtained in a case of eucaryote, by extracting mRNA from cell or tissue samples thereof and converting the labelled tNTP ("dNTP" means a deoxyribonucleotide in which the base is adenine (A), cytosine (C), guanine (G), or thymine (T)) as the base into cDNA by reverse transcription while incorporating the same. In a case of procaryote such as bacteria, total RNA is extracted since selective extraction of mRNA is difficult. In a case of using the genome as the specimen, it can be isolated from optional tissue samples excluding red cells. The optional tissues excluding the red cells are, for example, peripheral blood lymphocytes, skins, hairs, sperms, etc. The amount of mRNA required for hybridization for once is different depending on the amount of liquid or the labeling method and it is usually several µ g or less. There is also a method of using mRNA as an antisense RNA. As the labelled dNTP, use of a labelled dCTP is preferred in view of the chemical stability. Further, in a case where the polynucleotide as a probe immobilized on the solid support is an oligonucleotide, it is desirable that the molecular weight of the polynucleotide specimen is lowered.

For the purpose of examining the gene variant or polymorphism, the labelled polynucleotide specimen is generally obtained by conducting PCR for the target region in a reaction system containing a labelled primer or the labelled dNTP.

As a label, RI label and non-RI label (fluorescence method, biotin method, chemical emission method, etc.) are known and the fluorescence label is used preferably. The fluorescence label includes, for example, a CyDye such as Cy3 and Cy5, FITC, RITC, rhodamine, Texas red, TET, TAMRA, FAM, HEX, ROX, etc. and the radioactivity label includes, for example, α⁻³²P, γ⁻³²P, ³⁵S, etc.

Then, the labelled polynucleotide specimen is dissolved or dispersed in an aqueous medium such as SSC (saline-sodium citrate) to prepare an aqueous solution of the specimen. After spotting the aqueous solution on the solid support to which the polynucleotide is immobilized, it is incubated to conduct hybridization. Spotting can be practiced by distributing an aqueous solution of the specimen, for example, to a 96-hole or 384-hole plastic plate, etc. and dripping the solution by using a spotting device. The spotting amount of the aqueous solution is preferably within a range from 100 pL to 100 nL per spot. Incubation is conducted preferably at a temperature within a range from room temperature to 100°C and for a time within a range from 1 to 24 hours.

In a case of a micro-array for DNA analysis, while the amount of the specimen of the DNA fragment to be used can be extremely small, optimal conditions have to be set in accordance with the chain length and the kind of the specimen of the probe polynucleotide immobilized to the solid support upon hybridization. For the analysis of the gene expression, it is preferred to conduct hybridization for a long time so that also the gene of low expression can be detected sufficiently. On the other hand, for the detection of single base variant, the hybridization is preferably conducted in a short time. Thus, the probe polynucleotide on the solid support and the molecule having the base sequence complementary to the probe in the specimen of the labelled nucleic acid fragment are hybridized to form a hybrid.

After the completion of the hybridization, cleaning is conducted preferably by using a mixed solution of a surfactant solution and a buffer solution to remove the unreacted polynucleotide specimen. As the surfactant, sodium dodecyl sulfate is used preferably. As the buffer while a citrate buffer, phosphate buffer, borate buffer, tris buffer, good buffer, ect. can be used, the citrate buffer is used preferably.

Then, florescence signals from the hybrid formed on the solid support are detected by a detector. As the detector, a florescence scanning apparatus in which a florescence laser microscope, a cooled CCD camera and a computer are connected is used and the florescence intensity on the solid support can be measured automatically. Instead of the CCD camera, a co-focal or non-focal type laser may also be used. Thus, image data are obtained. From the obtained data, a target polynucleotide complementary to the probe polynucleotide immobilized on the solid support can be identified and, based thereon, a gene expression profile can be prepared or the base sequence of the polynucleotide specimen can be determined. Further, by utilizing a data analysis software or an external data base, more complicate analysis for gene variant, polymorphism, etc. can also be conducted. Alternatively, by providing plural kinds of polynucleotide specimens labelled by florescent substances different from each other as the specimens and using them simultaneously, comparison and quantitative determination for the expression amount can be conducted on one solid support.

### Example

The present invention is to be described by way of examples but the invention is not restricted to them.

### (Example 1) Preparation of Solid Support

Slide glass of 25 mm (width) × 75 mm (length) × 1 mm (thickness) was dipped in an aqueous polyallyl amine solution (0.1 g/l) to form an electrostatic layer. Then, a polyacrylic acid as a polybasic carboxylic acid was condensed to the amino group in the electrostatic layer under the presence of 0.1M 1-[3-(dimethylamino)propyl]-3-ethyl carbodiimide. Then, it was activated by being immersed for 30 min in an activating solution in which 0.1M 1-[3-(dimethylamino)propyl]-3-ethyl carbodiimide-hydrogen chloride and 20 mM of N-hydroxy succinic imide were dissolved in 300 ml of 0.1 M phosphate buffer (pH 6).

### (Example 2) Spotting of DNA Immobilizing Solution

Aqueous Cy3-labelled DNA solutions having the following compositions (a) to (e) were prepared respectively and spotted by using a spotter device (SPBIO2000, manufactured by Hitachi Software Engineering Co.) on the solid support prepared by Example 1.
(a) Cy3 fluorescence labelled DNA 0. 5 µg/µl, 1 × Solution TTM (manufactured by Takara Bio Co.), 10% glycerol.
(b) Cy3 fluorescence labelled DNA 0.5 µg/µl, 0.05M NaOH, 10% glycerol.
(c) Cy3 fluorescence labelled DNA 0.5 µg/µl, 10% isopropanol.
(d) Cy3 fluorescence labelled DNA 0.5 µg/µl, 1 × Arraylt (trademark: manufactured by TeleChem International Co.).
(e) Cy3 fluorescence labelled DNA 0.5 µg/µl, 10% diethylene glycol.

After spotting, the following procedures were conducted.
(1) The DNA-spotted solid support was placed in an oven set at 80°C and dried for 1 hour.
(2) The solid support was set to a holder.
(3) A cleaning solution (2 × SSC/0.2% SDS, room temperature) was charged in a 500 ml beaker in which the solid support set to the holder was immersed and cleaned for 15 min while being stirred by a stirrer.
(4) In the same manner, a cleaning solution (2 × SSC/0.2% SDS, 90°C) heated to 90°C was charged, to which the solid support set to the holder was immersed and cleaned for 5 min while being stirred by a stirrer.
(5) The solid support was rinsed by sterilized water.
(6) The solution on the surface of the solid support was blown off by a centrifugator.
(7) Fluorescence derived from Cy3 fluorescence labelled DNA immobilized to the solid support was measured by using FLA8000 (manufactured by Fuji Film Inc.).

The results are shown in Fig. 1

From the results shown in Fig. 1, in a case where the DNA solutions of the composition (a) to (d) were spotted, spots were irregular and the spot shape was not uniform such that the spot size was enlarged, spot was formed not uniformly, the spot shape was not circular, or the spot was blanked into a doughnut shape. On the contrary, it has been formed that in a case of dissolving DNA into an aqueous solution containing diethylene glycol and spotting the solution on the solid support, the spot shape was circular and spotting could be conducted with no blanking and in a uniform spot shape with no irregularity. Further, in view of the value of the fluorescence intensity, it can be seen that the total signal strength per spot is high and the signal strength per pixel is also high in a case of spotting by the method according to the invention. From the forgoing results, it has been found that the polynucleotide is immobilized firmly and uniformly on the solid support by the method of immobilizing the polynucleotide according to the invention.

### (Example 3)

Aqueous Cy3 labelled DNA solutions having the following compositions (f) to (i) were prepared respectively and spotted by using a spotter device (SPBIO2000, manufactured by Hitachi Software Engineering Co.) on the solid support prepared by Example 1.
(f) Cy3 fluorescence labelled DNA 5 µmol/l, 20% polyethylene glycol (molecular weight 300)
(g) Cy3 fluorescence labelled DNA 5 µmol/l, 10% dimethyl sulfoxide, 1% polyethylene glycol (molecular weight 300)
(h) Cy3 fluorescence labelled DNA 5 µmol/l, 1 × Solution I (trademark: manufactured by TaKaRa)
(i) Cy3 fluorescence labelled DNA 5 µmol/l, 3 × SSC (physiological saline, concentrated by 3 times)

After spotting, the following procedures were conducted.
(1) The DNA-spotted solid support was placed in an oven set at 80°C and dried for one hour.
(2) The solid support was set to a holder.
(3) A cleaning solution (2 × SSC/0.2% SDS, room temperature) was charged in a 500 ml beaker in which the solid support set to the holder was immersed and cleaned for 15 min while being stirred by a stirrer.
(4) In the same manner, a cleaning solution (2 × SSC/0.2% SDS, 90°C) heated to 90°C was charged, to which the solid support set to the holder was immersed and cleaned for 5 min while being stirred by a stirrer.
(5) The solid support was rinsed by sterilized water.
(6) The solution on the surface of the solid support was blown off by a centrifugator.
(7) Fluorescence derived from Cy3 fluorescence labelled DNA immobilized to the solid support was measured by using FLA8000 (manufactured by Fuji Film Inc.).

The results are shown in Fig. 2

From the results shown in Fig. 2, in a case where the DNA solution of the composition (h and i) were spotted, spots were irregular and the spot shape was not uniform such that the spot size was enlarged, spot was not formed uniformly, the spot shape was not circular or the spot was blanked into a doughnut shape. On the contrary, in a case (f or g) of dissolving the DNA into an aqueous solution containing polyethylene glycol (molecular weigh 300) by 20% or 1% and spotting the solution on the solid support, it was observed that the spot shape was circular and spotting could be conducted with no blanking and with a uniform spot shape with no irregularity. Further, in view of the value of the fluorescence intensity, it can be seen that the total signal strength per spot is high and the signal strength per pixel is also high in a case of spotting by the method according to the invention. From the forgoing results, it has been found that the polynucleotide is immobilized firmly and uniformly on the solid support by the method of immobilizing the polynucleotide according to the invention.

### Industrial applicability

According to the method of the present invention, since the polynucleotide can be spotted and immobilized on a solid support in a uniform spot shape with no irregularity in a circular shape and without blanking, data of high reliability with no scattering of the signal strength can be obtained in the detection of the polynucleotide by the micro-array.

## Claims

1. A method of spotting a solution containing a polyether or glycol and a polynucleotide on a solid support thereby immobilizing the polynucleotide on the solid support.

2. A method according to claim 1, wherein the solid support has a functional group capable of covalent bonding with the polynucleotide.

3. A method according to claim 2, wherein the solid support has an electrostatic layer for electrostatically attracting the polynucleotide on a substrate.

4. A method according to any one of claims 1 to 3, wherein the polynucleotide is DNA.

5. A method according to any one of claims 1 to 4, wherein the polyether or glycol is diethylene glycol or polyethylene glycol.

6. A solid support in which a polynucleotide is immobilized by the method according to any one of claims 1 to 5.

7. A method of detecting a target polynucleotide which includes hybridizing a labelled polynucleotide to a polynucleotide immobilized on a solid support by a method according to any one of claims 1 to 5, and reading a signal derived from the labelled polynucleotide.

8. A solution for immobilizing a polynucleotide **characterized by** containing a polyether or glycol.
